# EUROPEAN PATENT APPLICATION

(11) **EP 2 949 743 A1**
(43) Date of publication of application: **02.12.2015**
(21) Application number: 15168874.4
(22) Date of filing: 22.05.2015
(51) Int. Cl.: C12M 1/00, C12M 1/26

(54) **STIMULATION-RESPONSIVE MATERIAL AND CELL CULTURE CONTAINER MADE THEREOF**

(30) Priority: 30.05.2014 JP 2014111873
(71) Applicant: Hitachi, Ltd., Tokyo 100-8280 (JP)
(72) Inventor: Shibuya, Keisuke, Tokyo, 100-8280 (JP); Maruyama, Masashi, Tokyo, 100-8280 (JP); Tada, Yasuhiko, Tokyo, 100-8280 (JP)
(74) Representative: MERH-IP Matias Erny Reichl Hoffmann

(57) **Abstract**

A stimulation-responsive material comprises regions of pH-responsive polymer and regions of temperature-responsive polymer, the regions existing at different locations.

## Description

### BACKGROUND OF THE INVENTION

### 1. Field of the Invention

The present invention relates to a stimulation-responsive material and a cell culture container made thereof which permit one to maintain and control the environment for cell culture in a cell culture apparatus and to noninvasively recover normally cultured cells.

### 2. Description of the Related Art

Regenerative medicine includes a therapy which consists of collecting cells from a patient, proliferating the collected cells in vitro, inducing the cells to differentiate into cell tissues (if necessary), and returning the resulting cells to the affected part of the patient. For the stable proliferation and culture of cells in vitro, it is important to maintain constant and control the environment for culture and to noninvasively recover the resulting cells.

The quality of cells is affected by the environment of culture which varies depending on such factors as temperature, pH, dissolved oxygen, and dissolved carbon dioxide. Cell culture is usually performed by specialists' manual operation; however, the stable supply of cells needs automatic cell culture with a special apparatus. Either of manual operation and automatic cell culture involves a problem of difficulties with on-line pH monitoring. In manual operation it is only possible to maintain constant the temperature and CO₂ concentration in the incubator and it is impossible to perform on-line pH monitoring in the cell culture container. The apparatus for automatic culture also presents difficulties in keeping it sterile when attaching and calibrating a pH electrode.

There is a problem also in the recovery of cells, which is usually accomplished by peeling cells off the bottom of culture container with the help of enzyme such as trypsin. This peeling procedure causes damage to cells. To address this problem, there has recently been developed a new material for the bottom of culture container, which permits cells to peel off noninvasively by changing between hydrophilicity and hydrophobicity in response to temperature. (For instance, see JP-9-49830-A.)

For cells to be used as a medical material, it is necessary not only to control culture in an adequate environment but also to recover cells noninvasively. Unfortunately, there exists no culture container to meet such requirements.

### SUMMARY OF THE INVENTION

It is an object of the present invention to provide a stimulation-responsive material and a cell culture container made thereof, which permit one to detect pH change in the culture medium during culture and to noninvasively recover cultured cells in the form of cell sheet in the case of normal culture.

After extensive investigation to achieve the foregoing object, the present inventors found a new material for the bottom of culture container having the following characteristic properties. The new material is a polymer that coats the bottom of culture container. This polymer changes in hydrophilicity and hydrophobicity in response to pH and temperature independently on the bottom surface of culture container. With a normal pH value, the bottom surface remains hydrophobic and hence permits cells to adhere thereto and proliferate thereon. With an anomalous pH value beyond the control range, the bottom surface becomes hydrophilic and hence causes cells to peel off. After normal cell proliferation, the temperature is lowered beyond the culture temperature while the adequate pH value is being maintained. The lowering of temperature changes the bottom surface from hydrophobic to hydrophilic, thereby allowing the noninvasive recovery of cells. The foregoing finding led to the present invention.

In other words, the stimulation-responsive material according to the present invention consists of regions of pH-responsive polymer and regions of temperature-responsive polymer, which are arranged at different positions.

According to the present invention, the stimulation-responsive material and the cell culture container made thereof permit cultured cells to be noninvasively recovered without the help of enzyme in the case of culture in an adequate environment but permit cultured cells to peel off without recovery in the case of culture under an anomalous condition with pH values beyond the control range. This helps supply highly safe cells, particularly those for medical use.

### BRIEF DESCRIPTION OF THE DRAWINGS

Other objects and advantages of the invention will become apparent from the following description of embodiments with reference to the accompanying drawings in which:
Fig. 1 is a diagram showing the skeleton of the stimulation-responsive material;
Fig. 2 is a schematic diagram showing how cells adhere and peel off when they respond to stimulation;
Fig. 3 is a diagram showing the synthesis of temperature-responsive polylysine;
Fig. 4 is a diagram showing the synthesis of temperature-responsive polyglutamic acid;
Fig. 5 is a diagram showing the synthesis of temperature-responsive polyaspartic acid;
Fig. 6 is a diagram showing the temperature dependence of the stimulation-responsive material. The abscissa represents temperature and the ordinate represents light transmittance;
Fig. 7 is a diagram showing the pH dependence of the stimulation-responsive material. The abscissa represents pH and the ordinate represents light transmittance;
Fig. 8 is a diagram showing the procedure for immobilizing the stimulation-responsive material onto the culture plate; and
Fig. 9 is a schematic diagram showing the procedure for culturing a cell sheet.

### DESCRIPTION OF THE PREFERRED EMBODIMENTS

The following is a detailed description of the invention.

According to the present invention, the stimulation-responsive material is required to change in hydrophilicity and hydrophobicity independently from each other in response to pH and temperature. To meet this requirement, the stimulation-responsive material is composed of a pH-responsive polymer and a temperature-responsive polymer, which are arranged in different regions. Although the polymer illustrated below is made of polyamino acid, the polymer may also be a combination of different ones responsive to light, temperature, and pH. The resulting stimulation-responsive material would be responsive independently to two or more stimuli.

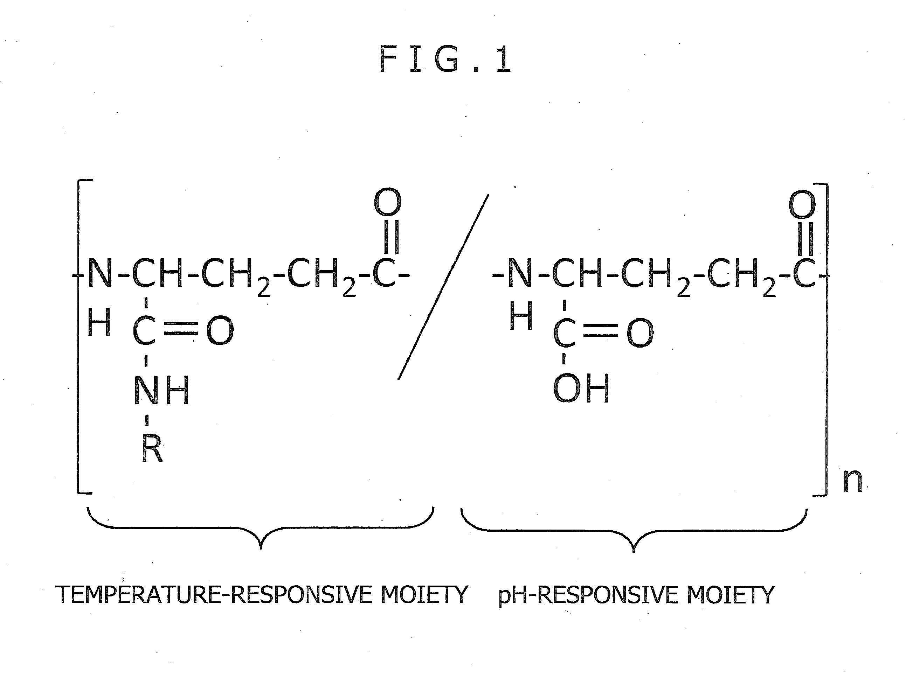

One material capable of responding to two kinds of stimuli has the skeleton as shown in Fig. 1. It has the moiety (structure 1) which changes in molecular structure upon stimulation with temperature and the moiety (structure 2) which changes in molecular structure upon stimulation with pH.

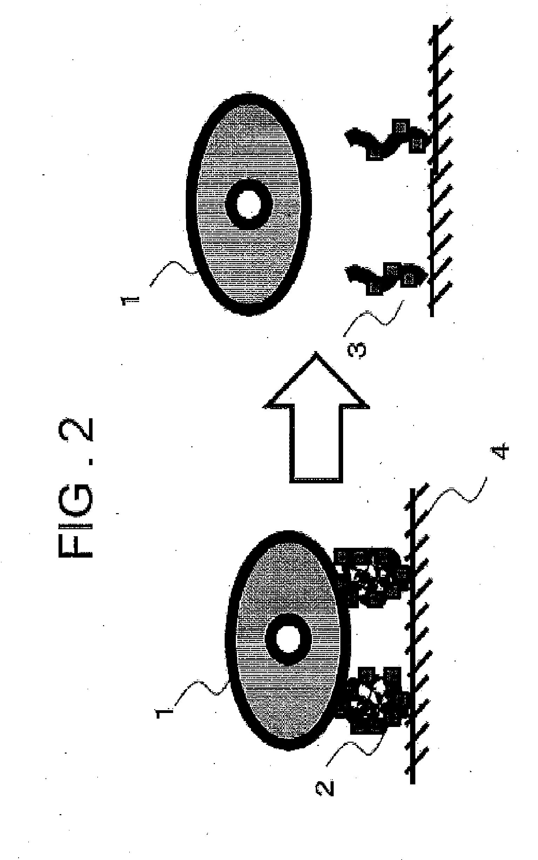

The temperature-responsive polymer falls under two categories: UCST type (upper critical soluble temperature) and LCST type (lower critical soluble temperature). The former is insoluble in solvents under the critical temperature but soluble in solvents above the critical temperature. The latter is soluble in solvents under the critical temperature but insoluble in solvents above the critical temperature. Also, the temperature-responsive polymer constituting the culture surface should preferably change from hydrophobic to hydrophilic as the result of response to stimulation when the cell sheet is peeled off. To this end, the cell culture step should preferably be performed under the condition that the polymer (as the temperature-responsive moiety) is soluble (with the polymer entirely hydrated) and the cell peeling step should preferably be performed under the condition that the polymer (as the temperature-responsive moiety) is insoluble (with the polymer dehydrated and aggregated due to great mutual reactions between molecules). This is illustrated in Fig. 2. Incidentally, the temperature-responsive polymer of LCST type can be made by introduction of hydrophobic side chains into the hydrophilic main chains.

The foregoing is true also for pH stimulation. That is, the cell culture step should preferably be performed under the condition that the polymer (as the pH-responsive moiety) is soluble (with the polymer entirely hydrated) and the cell peeling step should preferably be performed under the condition that the polymer (as the pH-responsive moiety) is insoluble (with the polymer dehydrated and aggregated due to great mutual reactions between molecules).

The stimulation-responsive material should preferably be prepared from polyamino acid produced by bacteria belonging to the genus streptomyces. The polyamino acid is exemplified by polylysine, which is a tissue-derived biomaterial consisting of amino acids. It is inexpensive and highly capable of chemical modification and introduction into the support surface (owing to amino groups (-NH₂) therein). Thus it is suitable for use as the basic skeleton of the temperature-responsive moiety.

According to the present invention, the moiety as structure 1 is a polymer having the lower critical soluble temperature. This polymer includes the following examples:
polymers of N-substituted (meth)acrylamide derivatives, such as N-n-propylacrylamide, N-isopropylacrylamide, N-ethylacrylamide, N,N-dimethylacrylamide, N-acryloylpyrrolidine, N-acryloylpiperidine, N-acryloylmorpholine, N-n-propylmethacrylamide, N-isopropylmethacrylamide, N-ethylmethacrylamide, N,N-dimethylmethacrylamide, N-methacryloylpyrrolidine, N-methacryloylpiperidine, and N-methacryloylmorpholine;
polyoxyethylenealkylamine derivatives, such as hydroxypropylcellulose, partially acetified product of polyvinyl alcohol, polyvinyl methyl ether, (polyoxyethylene-polyoxypropylene) block copolymer, and polyoxyethyleneraulylamine;
polyoxyethylene sorbitan ester derivatives, such as polyoxyethylene sorbitan laurate;
(Polyoxyethylene alkylphenyl ether) (meth)acrylates, such as (polyoxyethylene nonylphenyl ether) acrylate, and (polyoxyethylene octylphenyl ether) methacrylate; and
polyoxyethylene (meth)acrylate ester derivatives, such as (polyoxyethylene alkyl ether) (meth)acrylates, e.g., (polyoxyethylene lauryl ether) acrylate and (polyoxyethylene oleyl ether) methacrylate.

The foregoing polymers may be used in the form of copolymer composed of at least two kinds of monomers. Such copolymers include the one which is composed of N-isopropylacrylamide and N-t-butylacrylamide.

The polymer containing (meth)acrylamide derivatives may be used in the form of copolymer composed of the polymer and monomers copolymerizable with the polymer, with the monomers being used in such an amount that the resulting copolymer has the lower critical soluble temperature.

According to the present invention, it is desirable to use any polymer composed of at least one species of monomer selected from the group consisting of N-n-propylacrylamide, N-isopropylacrylamide, N-ethylacrylamide, N,N-dimethylacrylamide, N-acryloylpyrrolidine, N-acryloylpiperidine, N-acryloylmorpholine, N-n-propylmethacrylamide, N-iso-propylmethacrylamide, N-ethylmethacrylamide, N,N-dimethylmethacrylamide, N-methacryloylpyrrodine, N-methacryloylpiperidine, and N-methacryloylmorpholine. Another desirable one is a copolymer of N-isopropylacrylamide and N-t-butylacrylamide.

The moiety as structure 2 may be not only a carboxyl group but also phosphoric acid group or any functional group of sulfonyl, amino, etc. For example, it may be a polymer composed of monomers having such dissociable groups as (meth)acrylic acid, maleic acid, styrenesulfonic acid, 2-acrylamide-2-methylpropanesulfonic acid, phosforylethyl (meth)acrylate, aminoethyl methacrylate, aminopropyl (meth)acrylamide, and dimethylaminopropyl (meth)acrylamide. It may also be a copolymer composed of the monomers (having dissociable groups) and such additional monomers as (meth)acrylate esters (e.g., methyl (meth)acrylate, ethyl (meth)acrylate, and butyl (meth)acrylate), vinyl esters (e.g., vinyl acetate and vinyl propionate), vinyl compounds (e.g., styrene, vinyl chloride, and N-vinylpyrrolidone), and (meth)acrylamides. The amount of the additional monomers should be small enough for the resulting copolymer to have adequate pH responsiveness.

The following is a description of the method for preparing a stimulation-responsive material with polyamino acid and the application thereof to culture.

### (1) Preparation of a stimulation-responsive material with polyamino acid

### • Preparation of a temperature-responsive polyamino acid

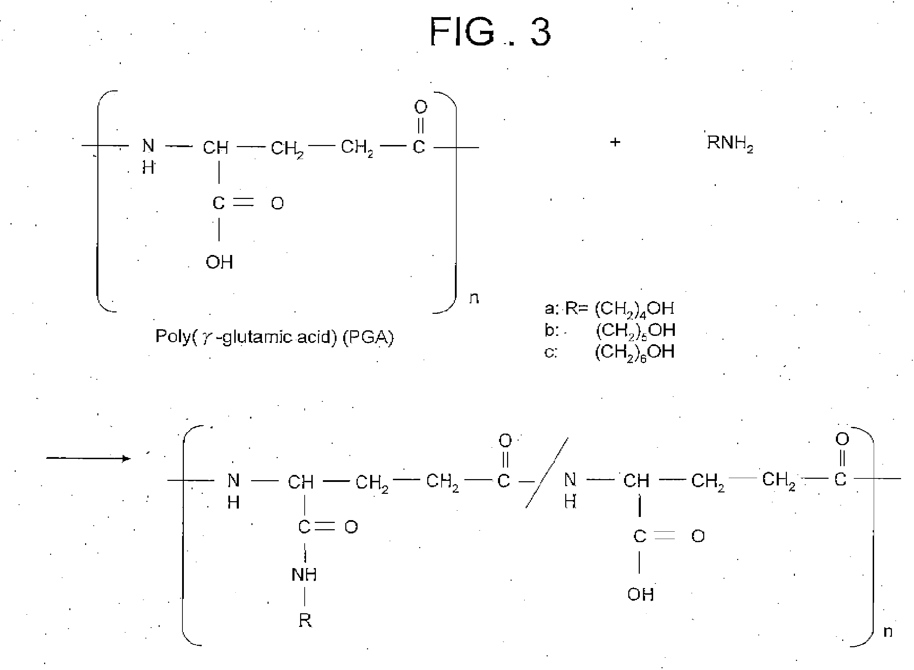

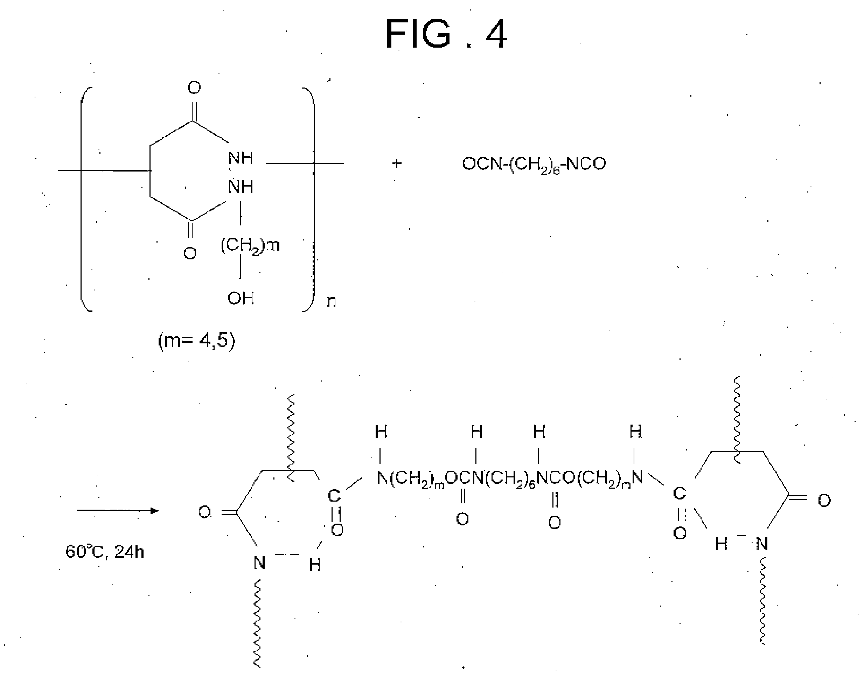

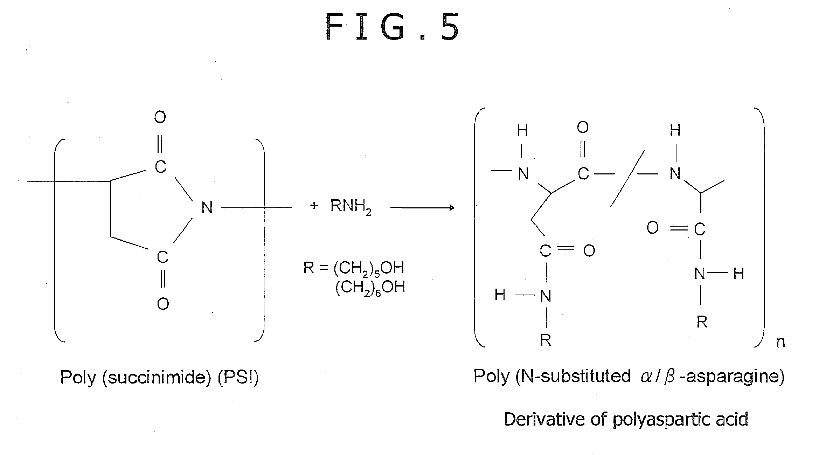

Known among temperature-responsive polyamino acids are those based on polylysine, polyglutamic acid, and polyaspartic acid. They are synthesized according to the scheme shown in Figs. 3 to 5.

### (i) Preparation of temperature-responsive polylysine

In 4 mL of distilled water or PBS (pH 5.8) (held in a glass container) were dissolved 290 mg of WSC, 170 mg of NHS, and 130 µL of valeric acid. To the resulting solution (cooled to 4°C) was added 140 mg of polylysine. The reactants underwent reaction overnight with stirring. (This reaction may be replaced by the one which is accomplished for 2 hours at 37°C.) The reaction product was purified and separated by dialysis to remove unreacted matter, which was performed through a dialysis membrane for the cut-off molecular weight of 2000 (Spectra/Pore@) by stirring in pure water at room temperature for 24 hours. (ii) Preparation of temperature-responsive polyaspartic acid

In a separable flask (equipped with a stirrer and thermometer) was placed 34 g of N,N-dimethylformamide (DMF). In the DMF was dissolved 9.7 g (0.1 mol) of poly(succinimide) (PSI). To the resulting solution were added 6.5 g (0.035 mol) of dodecylamine (LA) and 4.9 g (0.065 mol) of 2-methoxyethylamine (MOE), followed by reaction at 70°C for 6 hours. The resulting solution was poured into a large amount of acetonitrile, followed by filtration to recover precipitates (19.4 g with a yield of 92%). The recovered precipitates were dried below 60°C for 24 hours.

### (2) Evaluation of the stimulation-responsive material for responsiveness

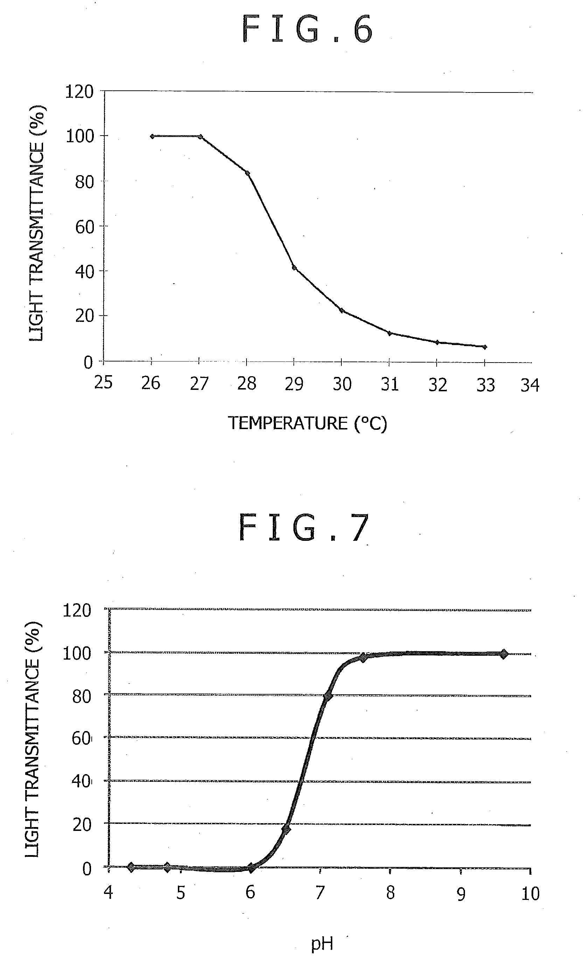

The resulting polylysine was tested for LCST, which is defined as the temperature at which a 1 wt% solution (in PBS 7.4) gives a light transmittance of 90% measured by a temperature-variable UV-visible absorption spectrometer. The results are shown in Fig. 6. It is to be noted that the polylysine gives the LCST at about 28°C. This means that the polylysine is hydrophobic at 37°C (suitable for cell culture) and becomes hydrophilic below 27°C (suitable for cell peeling).

The same procedure as above was repeated except that the light transmittance was measured at 37°C, with the solvent pH varied. The results are shown in Fig. 7. It is to be noted that the polylysine changes from hydrophilic to hydrophobic in response to pH stimulation.

### (3) Immobilization of the stimulation-responsive material onto a plate

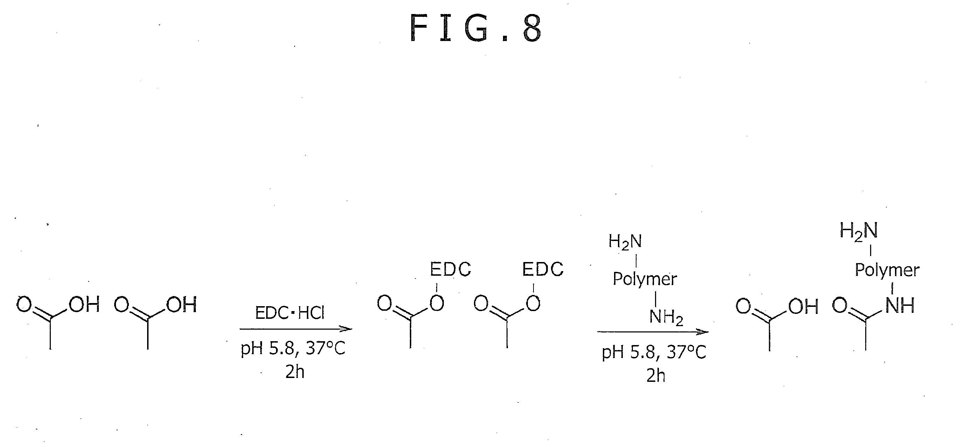

A 6-well microplate having carboxyl groups on its surface was reacted with PBS 5.8 solution of EDC•HCl (1.0 wt%, 10 mL) at 37°C for 2 hours. The resulting surface-activated microplate was washed with PBS 5.8 and then reacted with PBS 5.8 solution of polymer (0.001 to 1 wt%, 10 mL) at 37°C for 2 hours. After washing with PBS 7.4, there was obtained a polymer-supporting surface. (See Fig. 8.)

### (4) Application to cell culture

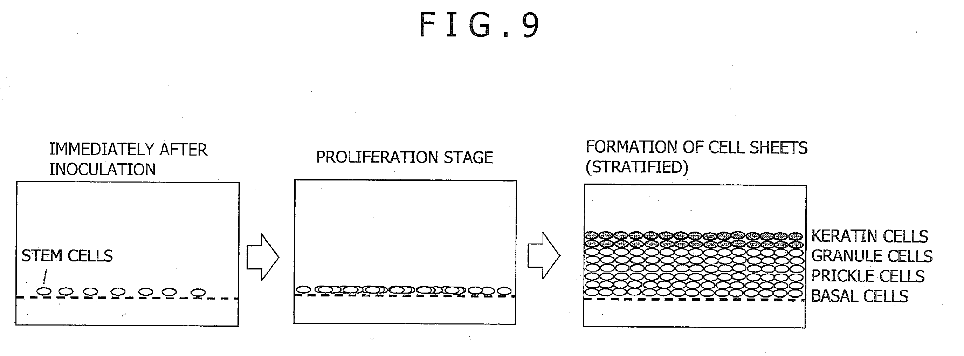

The following is a description of the case in which the above-mentioned stimulation-responsive material was applied to cultivation of human oral cells or human corneal cells to make a cell sheet. Human oral or corneal cells are adherent cells, and they initially proliferate in the undifferentiated state on the culture surface of the culture container. As they reach the confluent state, they proliferate in the form of layer. At this stage, the cells begin differentiation. When differentiation has completed, the cells are recovered. (See Fig. 9.) Although cell culture is usually performed in a cell culture container whose surface is made of plastics or NIPPAm, cell culture in the present invention was performed in a cell culture container whose surface is coated with the stimulation-responsive material.

### [Culture of cells to make a cell sheet]

### (i) Cells and culture medium

Human oral cells (from ScienCell) and human corneal cells (from Invitrogen) were used for the experiment. Proliferation of these cells was performed by using the special culture medium (free of serum) for each of them. The stratification of cells was accomplished by using KCM for both of them.

### (ii) Method of culture

The 6-well microplate coated with the stimulation-responsive material was inoculated with human oral cells or human corneal cells. Culture was performed in an incubator (37°C, 5% CO₂, >95% humidity) by using DMEM/F12 medium containing glucose. Culture was continued, with the medium renewed once every three or four days, until proliferation reached the confluent state. At this stage, the medium was replaced by the differentiation inducing medium (KCM medium) for differentiation. With the KCM medium renewed once every three or four days, the proliferated cells were stratified. In this way there was obtained a cell sheet.

### (iii) Recovery of cells

The normally cultured cells were recovered by lowering the temperature from 37°C to 20°C.

### [Evaluation of culture]

It was found that cells proliferated without peeling in the case of culture with the medium kept at pH 6.8-7.6. By contrast, it was also found that cells peeled off from the culture surface in the case where the medium decreased in pH below 6.8 due to contamination with bacteria. The same was also true in the case where the medium decreased in pH below 6.8 due to the accumulation of lactic acid secreted from cells which occurred due to delayed medium replacement.

On the other hand, in the case where the environment of culture remained unaffected unlike the foregoing case, cells proliferated and constituted a cell sheet due to differentiation induction. The resulting cell sheet was easily peeled off from the culture surface when it was given the temperature stimulation (or change from 37°C to 20°C).

### [Exploitation in Industry]

The method and apparatus according to the present invention, which is intended for stratification and/or judgment of the degree of differentiation, will be applicable to the cells for regenerative medicine which need culture processes under good quality control and transplantation at adequate timing. They will also be used to monitor cells during cell culture for regenerative medicine.

Features, components and specific details of the structures of the above-described embodiments may be exchanged or combined to form further embodiments optimized for the respective application. As far as those modifications are apparent for an expert skilled in the art they shall be disclosed implicitly by the above description without specifying explicitly every possible combination.

## Claims

1. A stimulation-responsive material comprising regions of pH-responsive polymer and regions of temperature-responsive polymer, the regions existing at different locations.

2. The stimulation-responsive material as defined in Claim 1, which is **characterized in** having a skeleton of polyamino acid whose side chains are chemically modified with hydrophobic functional groups.

3. The stimulation-responsive material as defined in Claim 1 or 2, wherein the temperature-responsive polymer is that polymerized from at least one species of monomer selected from N-n-propylacrylamide, N-isopropylacrylamide, N-ethylacrylamide, N,N-dimethylacrylamide, N-acryloylpyrrolidine, N-acryloylpiperidine, N-acryloylmorpholine, N-n-propylmethacrylamide, N-isopropylmethacrylamide, N-ethylmethacrylamide, N,N-dimethylmethacrylamide, N-methacryloylpyrrolidine, N-methacryloylpiperidine, and N-methacryloylmorpholine.

4. The stimulation-responsive material as defined in at least one of the claims 1-3, wherein the pH-responsive polymer is that polymerized from monomers containing at least one species of group selected from carboxyl group, phosphoric acid group, sulfonyl group, and amino group.

5. The stimulation-responsive material as defined in at least one of the claims 1-4, wherein the pH-responsive polymer is that polymerized from at least one species of monomer selected from (meth)acrylic acid, maleic acid, styrenesulfonic acid, 2-acrylamide-2-methylpropanesulfonic acid, phosforylethyl (meth)acrylate, aminoethyl methacrylate, aminopropyl (meth)acrylamide, and dimethylaminopropyl (meth)acrylamide.

6. The stimulation-responsive material as defined in at least one of the claims 1-5, which changes from hydrophobic to hydrophilic at a pH value lower than 6.8.

7. The stimulation-responsive material as defined in at least one of the claims 1-6, which changes from hydrophobic to hydrophilic at a pH value higher than 7.6.

8. The stimulation-responsive material as defined in at least one of the claims 1-7, which is hydrophobic at a temperature no lower than 30°C and becomes hydrophilic at a temperature no higher than 30°C.

9. A cell culture container which has a culture surface onto which are immobilized the stimulation-responsive molecules of the stimulation-responsive material as defined in at least one of the claims 1-8.

10. The cell culture container as defined in Claim 9, wherein the stimulation-responsive molecules are immobilized through covalent bonding.

11. The cell culture container as defined in Claim 10, wherein the covalent bonding is amide bonding.
